# EUROPEAN PATENT APPLICATION

(11) **EP 3 954 358 A1**
(43) Date of publication of application: **16.02.2022**
(21) Application number: 19924082.1
(22) Date of filing: 11.04.2019
(51) Int. Cl.: A61K 8/97, A61K 8/9789, A61K 8/44, A61K 8/60, A61K 8/73, A61Q 19/00, A61Q 19/08

(54) **COSMETIC COMPLEX AND USES THEREOF**

(71) Applicant: Natura Cosméticos S.A., 05106-000 São Paulo SP (BR)
(72) Inventor: PEREIRA SANTOS, Camila, 05106-000 São Paulo - SP (BR); ZIMBARDI, Daniela, 05106-000 São Paulo - SP (BR); DE MIRANDA CHAVES VASQUEZ PINTO, Luciana, 05106-000 São Paulo - SP (BR); DO NASCIMENTO, Selma, 05106-000 São Paulo - SP (BR); ARANDAS MONTEIRO E SILVA, Silas, 05106-000 São Paulo - SP (BR); LIMA GUSMÃO, Edjane, 05106-000 São Paulo - SP (BR); ARRUDA COSTA, Andrea, 05106-000 São Paulo - SP (BR)
(74) Representative: Simmons & Simmons
(86) International application number: PCT/BR2019/050133
(87) International publication number: WO 2020/206514

(57) **Abstract**

The present invention describes a complex consisting of the association of *Fevillea trilobata* crude seed oil, trehalose and sodium hyaluronate, as well as cosmetic products, methods and uses to provide improved hydration and preserve the natural and healthy balance of the skin microbiota. The present invention is within the field of cosmetic science, more precisely it refers to cosmetic preparations to treat the skin.

## Description

### Field of the invention

The present invention describes a complex consisting of the association of *Fevillea trilobata* crude seed oil, trehalose and sodium hyaluronate, as well as cosmetic products, methods and uses to provide improved hydration and preserve the natural and healthy balance of the skin microbiota. The present invention is within the field of cosmetic science, more precisely it refers to a complex for use in cosmetic preparations for active hydration and preservation of the skin microbiota.

### Background of the invention

The skin is the largest and most visible organ in the human body and plays a fundamental role in maintaining life. It performs multiple vital functions to the body, and its role in protecting the body against fluid loss and preventing the penetration of foreign substances into the body has to be highlighted.

The skin is also an ecosystem composed of about 2 m² diverse habitats, with abundant folds, invaginations and specialized niches that can support a wide range of microorganisms such as bacteria, fungi, mites and viruses. These microorganisms comprise the so-called skin flora and, like the skin itself, they play a key protective role, as they contribute to the skin integrity and immune homeostasis.

Currently, both variations that change the composition of the skin layers and variations in the balance of the skin flora are known to cause damage to the skin which, in addition to causing discomfort, may also not be visually pleasing. Thus, there is a need to develop topical cosmetic compositions that contribute, at the same time, to the regulation of the skin morphology and the preservation of the balance of the microbial flora.

Stratum corneum is the skin region that contacts the outside environment, being the outer layer of the epidermis. Characteristics of the stratum corneum are critically related to its composition, being represented by proteins (75-80%), lipids (5-15%) and other constituents (5-10%). When these components are not properly balanced, the skin ability to hold water is reduced and the skin becomes more susceptible to the influence of environmental factors, causing an impaired barrier.

Endogenous factors due to aging or, for example, hormonal influence, and exogenous factors such as chronic exposure of the skin to dry environments, UV rays, wind, chemicals, etc., are factors that interfere with the skin ability to hold water. As a result, poorly hydrated skin (dry skin) lacks shine and flexibility, having a rough appearance and abnormal desquamation.

This type of skin requires specific care, since the lack of hydration can compromise the skin integrity.

Numerous moisturizing cosmetic compositions aim to be alternatives to solve the issue of dry skin. It is even recommended that adults use moisturizing compositions all over their skin at least once a day.

Traditional skin moisturizing products generally include ingredients that typically act through two well-known or passive mechanisms: occlusion and wetting. Hydration by occlusion is promoted by ingredients that form a lipophilic film that makes water loss more difficult, hence increasing water retention in the stratum corneum. Wetting hydration, in turn, is promoted by ingredients that retain water from the cosmetic composition, the atmosphere and the skin, on the skin surface through a hydrophilic film formed on the stratum corneum, hence increasing water retention on the skin surface.

On the other hand, many moisturizing cosmetics for topical use do not take into account that the skin is an ecosystem having a delicate balance between the host and microorganisms, therefore, they contain ingredients that hinder the development of beneficial microorganisms for healthy skin.

The skin is a niche for several microorganisms such as viruses, bacteria, fungi and mites that cover the skin surface and reside in the skin appendages that include hair follicles, sebaceous glands and sweat glands. These different microorganisms constitute a natural and healthy flora and provide vital functions to the body, protecting the skin from invasion of pathogenic or harmful organisms.

On the skin surface, bacteria such as those from the phylum *Proteobacteria* and from the genus *Staphylococcus* form communities that are deeply interconnected with each other and with other microorganisms. Commensal fungi such as *Malassezia spp.* grow both as branched filamentous hyphae and as individual cells. Skin mites, such as *Demodex folliculorum* rum and *Demodex brevis* are some of the smallest arthropods and live inside or near the hair follicles.

When there is an imbalance of the natural and healthy skin flora, the skin can suffer the invasion or manifestation of pathogenic bacteria, which can cause stress and inflammation, redness, itching, scarring, erythema and other reactions, leading to loss of the original skin radiance, while at the same time causes more skin problems and even skin aging.

The flora imbalance can be caused by the use of topical cosmetic compositions that contain ingredients that are harmful to the natural and healthy balance of the skin microbiota. Such compositions, therefore, can result in skin disorders or infections.

It is difficult to modify a cosmetic composition without changing its ability to satisfactorily play its key role, for example, hydration. On the other hand, it is a necessary task for the improvement of cosmetic compositions.

In the search for the state of the art in scientific and patent literature, the following documents dealing with the subject matter were found:

Document CN103462881 describes a cosmetic composition intended to promote the balance of skin microbiota.

Further, document CN103462881 comments on the need to maintain the skin microbiota to avoid skin damage, for example, growth of pathogenic microorganisms, due to an unbalanced microbiota. In fact, the document already describes that this need is much debated in modern cosmetics, being one of the factors that led to the replacement of antibiotics, hormones and other compounds that can be harmful to the balance of the natural microbiota when present in topical cosmetic compositions.

In detail, the composition described in the Chinese document has the following components: glycerin, butylene glycol, hamamelis extract, allantoin, EDTA trisodium, glycerin, purslane extract, alpha-glucan, oligosaccharide, xylitol, wicker extract, fructooligosaccharide, vitamin B5, Asian centella extract, rice bran extract, yeast extract, betaine, polyglutamic acid, sodium hyaluronate, beta-glucan, trehalose, potassium glycyrrhizinate, urea, sodium taurate, radish root fermentation product filter liquor and *leuconostoc.*

Document BR 11 2018 073287 refers to a cosmetic skin repairing composition, comprising an oil selected from *Bertholletia excelsa* or *Fevillea trilobata* and cosmetically acceptable excipients. The composition is said to provide an anti-aging treatment, including reducing wrinkles and promoting the skin regenerative capacity.

Furthermore, the Brazilian document describes that the composition acts by modulating cellular processes involved in skin repair, particularly in mechanisms of skin regeneration and healing, including FGF-2, IL-1B, IL-6, IL-8, IL- 10, IL-12, cell migration and proliferation associated with skin barrier construction and elastase activity (MMP-12).

Thus, it is understood that none of the documents presented above anticipate the aspects of the present invention, since they do not describe a cosmetic complex, which has a technical effect on the hydration and/or maintenance of the skin microbiota.

Thus, it is understood that, in view of the state of the art found, the problem related to the need to provide cosmetic compositions that enable the production of cosmetics having improved hydration efficiency and that aid to maintain the skin flora still exists.

### Summary of the invention

The present invention refers to a cosmetic complex that enables skin self-hydration, while helping to maintain the skin microbiota.

Thus, in a first aspect, the invention relates to a cosmetic complex comprising an association of the following ingredients: *Fevillea trilobata* seed crude oil, trehalose and sodium hyaluronate.

In other aspects, the invention relates to the use of the complex in accordance with the present invention, particularly in the form of a cosmetic product that is useful for moisturizing the skin while preserving the natural and healthy skin microbiota, as well as related methods.

These and other aspects of the invention will be immediately valued by those skilled in the art and by companies with interest in the segment, and will be described in sufficient detail to be reproduced in the description below.

### Brief description of the figures

In order to better define and clarify the present invention, the instant figures are presented:
Figure 1 shows the comparison between complex 1 (association of 0.5% fevillea crude oil, 3% trehalose and 0.25% sodium hyaluronate, by weight relative to the total weight of the placebo) and a placebo composition in terms of the expression of genes related to active skin hydration.
Figure 2 shows the comparison of complex 2 (association of 0.5% fevillea crude oil, 3% trehalose, 0.25% sodium hyaluronate and 2% betaine, by weight relative to the total weight of the placebo) with a placebo composition, in terms of the expression of genes related to active skin hydration.
Figure 3 shows the comparison of complex 1, complex 2 and a placebo relative to S. *epidermidis* viability kinetics.
Figure 4 shows the comparison of complex 1, complex 2 and a placebo relative to S. *aureus* viability kinetics.

### Detailed description of the invention

The present invention describes a cosmetic complex comprising the association of specific cosmetic ingredients, which complex enables skin hydration and aids in the maintenance of the skin microbiota.

For purposes of the present invention the term "Cosmetic Complex" refers to a mixture/association of at least three cosmetically acceptable active ingredients, particularly in the absence of cosmetic carrier excipients.

In the present invention the term "hydration" can be designated as "active hydration", "bioactive hydration", "biological hydration", "biodynamic hydration", "intelligent hydration". The hydration described herein stimulates the endogenous and natural mechanism of the skin, being able to maintain hydration at optimal levels for longer periods.

In the present invention, the term "maintenance of the skin microbiota" can also be understood as "preservation of the skin microbiota" or "microbiota balance", being defined as an ability to enable the presence of a natural and healthy skin microbiota. Furthermore, the term "skin microbiota" can also be understood as "skin flora".

The present invention differs from the state of the art in many ways. For example, the present invention is a complex containing a few ingredients, which are essential the skin hydration and preservation of the skin microbiota. The present invention further uses a *Fevillea* seed crude oil among its active ingredients and the state of the art neither teaches nor suggests associating such oil with the other complex components, which are essential, when combined, to trigger the technical effects observed.

Among the advantages of the present invention, in a non-exhaustive manner, is the promotion of improved active skin hydration and, simultaneously, preservation of the skin natural and healthy microbiota.

Ingredients of the complexes must be at least *Fevillea trilobata,* trehalose and sodium hyaluronate. Preferably, the ingredients of the complex should be *Fevillea trilobata,* trehalose, sodium hyaluronate and betaine.

Furthermore, the state of the art suggests using microorganisms or their metabolite derivatives to trigger a preserving effect on the skin microbiota, the present invention does not use this type of ingredient.

In a first aspect, the invention relates to a complex comprising at least an association of *Fevillea trilobata,* trehalose and sodium hyaluronate.

*Fevillea* crude oil is a product from the Brazilian biodiversity extracted from the seeds of *Fevillea trilobata* plant. It is an oil rich in punic acid or conjugated trienes (conjugated C18:3), beta-sitosterol, gamma-tocopherol and it further contains bioactive compounds such as alkaloids, terpenes, glycosides and tannins. On the skin, it stimulates the natural mechanisms for the production of hyaluronic acid, increasing water levels in the deeper layers of the skin (dermis). In addition, the oil also stimulates human skin cells to produce, by natural synthesis, elastin, filaggrin, ELOVL3 and claudin I.

*Fevillea's* ability to enhance the natural synthesis can be identified, for example, through an immunofluorescence test in an ex *vivo* skin model.

Trehalose is a biotechnological active of plant origin. The ingredient has a prebiotic function as it works to maintain and balance the skin microorganisms, helping to strengthen the skin barrier. Furthermore, it is a skin conditioning and humectant agent.

Sodium hyaluronate is a biotechnological active of natural origin. Its roles are the ability to increase water levels on the skin surface, acting as a skin conditioner or moisturizer.

Preferably, the complex comprises from about 7% to about 28% w/w *Fevillea trilobata* seed oil crude, from about 13% to about 76% w/w trehalose, and from about 4% to about 80% w/w sodium hyaluronate.

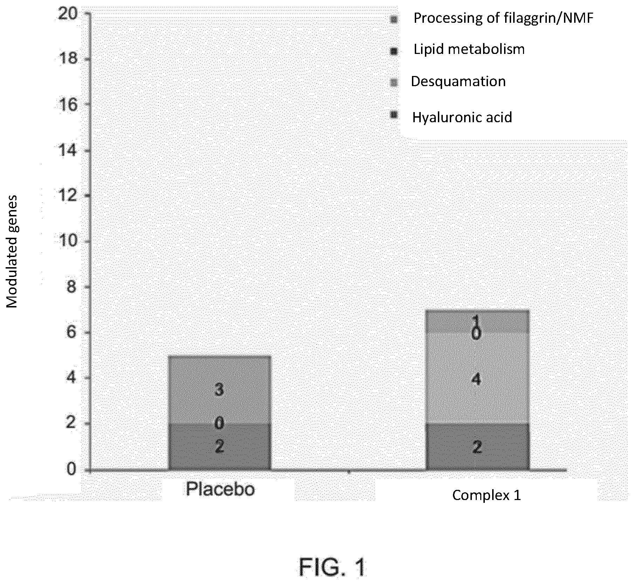

In one embodiment of the invention, the complex comprising *Fevillea trilobata* crude seed oil, trehalose and sodium hyaluronate, in the respective ratio of about 2:1:12, was designated complex 1 and assessed for its ability to act in "active hydration", that is, involving gene expression modulation, and for its ability to preserve skin microflora balance. To evaluate the activities, complex 1 was compared with a placebo and the results are shown in Figures 1, 3 and 4.

Even more preferably, the complex involves the use of an amino acid, betaine. In addition to betaine, which is preferred, the complex quantitatively contemplates the following ingredients: about 8% to about 17% *Fevillea trilobata* crude seed oil, about 28% to about 52% trehalose, about 4% to about 17% sodium hyaluronate, and about 34% to about 43% betaine.

Betaine can be obtained, for example, from sugar beet. Betaine improves the skin barrier, increases the number of natural (or endogenous) molecules that retain water on the surface layers and increases hydration.

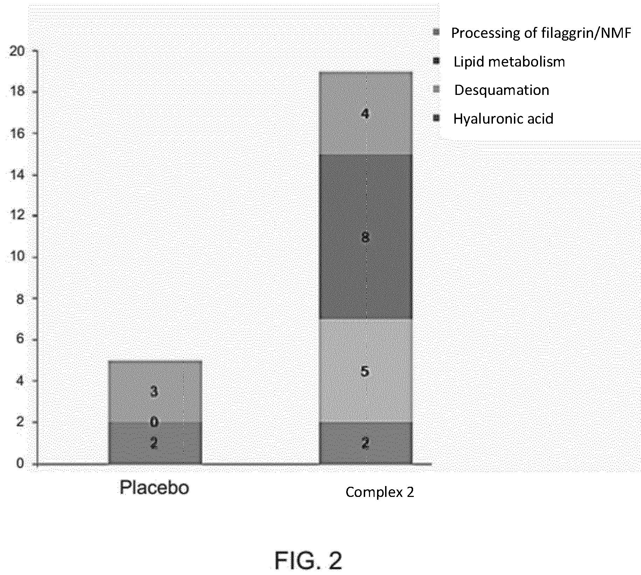

In another embodiment of the invention, the complex comprising *Fevillea trilobata* seed crude oil, trehalose, sodium hyaluronate and betaine in the respective ratio of about 2:12:1:8. In this embodiment, the complex is designated complex 2, and its ability to act in "active hydration", that is, involving gene expression modulation and in relation to preserving the balance of the skin microflora was evaluated. In comparison with a placebo formulation, it was noted that complex 2 has a positive effect on the analyzed activities, see figures 2, 3, 4.

In the present invention, the complex is able to stimulate five skin self-hydration mechanisms, namely: (1) stimulation of natural skin hydration factors (NMF), (2) stimulation of skin barrier lipids, (3) stimulation of essential proteins, (4) skin barrier cell renewal, (5) stimulation of endogenous hyaluronic acid.

In a second aspect, the invention relates to the use of the cosmetic complex as defined in the first aspect for skin hydration.

In a third aspect, the invention relates to the use of the cosmetic composition of the first aspect to preserve the skin microbiota.

In a fourth aspect, the invention relates to the use of the complex as defined in the first aspect in the manufacture of a topical cosmetic product to moisturize the skin.

In a fifth aspect, the invention relates to the use of the complex as defined in the first aspect in the manufacture of a topical cosmetic product to preserve the skin natural microbiota.

In a sixth aspect, the invention relates to a topical cosmetic product for hydrating and preserving the skin natural microbiota, which product contains the cosmetic complex as defined in the first aspect.

In a seventh aspect, the invention relates to a method to hydrate and preserve the skin natural microbiota, characterized in that it comprises applying the cosmetic complex onto the skin.

### Examples

The examples shown herein are intended only to exemplify one of the numerous ways of carrying out the invention, without limiting its scope.

The comparative tests presented below will be compared to a placebo topical cosmetic composition. By placebo is meant that the composition will not contain any of the ingredients of the cosmetic complex of the present invention.

The placebo, as described in table 1, is a traditional moisturizer mainly comprising the following ingredients: water, capric/caprylic triglyceride, sodium gluconate, penthyleneglycol, propanediol and sorbitol

**Table 1 - Placebo composition**

| Compound | Minimum Concentration (%) | Maximum Concentration (%) |
|---|---|---|
| Alpha-isomethyl ionone | 0.002 | 0.005 |
| Ammonium acrylomethyldimethyltaurate copolymer | 0.4 | 2 |
| Water | qsp | qsp |
| Capric/caprylic triglyceride | 1 | 3 |
| Carbomer | 0.3 | 0.6 |
| CI 42090 | 0.00006 | 0.0001 |
| Citronellol | 0.005 | 0.02 |
| *Conobea scoparioides* leaf oil | 0.001 | 0.003 |
| Dimethicone | 7 | 15 |
| Glycerin | 7 | 15 |
| Hydroxyacetophenone | 0.3 | 0.6 |
| Perfume | 0.1 | 0.3 |
| Pentylene glycol | 7 | 15 |
| Phenoxyethanol | 0.5 | 0.8 |
| Polyglyceryl-2 Sesquiisostearate | 0.04 | 0.1 |
| Polymethylsilsesquioxane | 1 | 3 |
| Propanediol | 3 | 7 |
| Sodium carbonate | 0.0003 | 0.0006 |
| Sodium Chloride | 0.00001 | 0.00004 |
| Sodium gluconate | 0.05 | 0.15 |
| Sodium hydroxide | 0.07 | 0.15 |
| Sodium Sulfate | 0.000004 | 0.000007 |
| Sorbitol | 0.1 | 0.3 |
| Tocopherol | 0.3 | 0.7 |
| Compound | Minimum Concentration (%) | Maximum Concentration (%) |
| Trilaureth-4 Phosphate | 0.05 | 0.1 |

Also, the results presented herein compared the placebo with complexes 1 and 2. For exemplary purposes of the present invention, complex 1 comprises *Fevillea trilobata* crude seed oil, trehalose and sodium hyaluronate at a 2:1:12 ratio. For exemplary purposes, complex 2 comprises *Fevillea trilobata* seed crude oil, trehalose, sodium hyaluronate and betaine at a 2:12:1:8 ratio.

### Example 1- Results of active hydration assessment

The result was based on the definition of active hydration concept, that is:
- Stimulation of the adequate and balanced removal of dead cells from the skin surface / renewal of skin barrier cells;
- Stimulation of the molecule that retains water on the surface such as NMF (natural hydration factors) (2) mineral salts and lipids (3).
- Stimulation of molecules that retain water in the deeper layers of the skin (dermis) such as hyaluronic acid (4);
- Stimulation of essential proteins to maintain the skin fluid balance (5).

Active hydration for the placebo, complex 1 and complex 2 was calculated and demonstrated in Figures 1 and 2 by evaluating the expression of genes involved in endogenous skin hydration or self-hydration. The results demonstrate that the placebo composition was not able to provide skin cell renewal; it was not able to stimulate lipids. Complex I had a technical effect, as it promoted a balanced skin desquamation. Complex II had an even superior technical effect, as it stimulated all mechanisms of skin self-hydration, or active hydration, such as balanced desquamation, lipid stimulation and metabolism, NMF stimulation and hyaluronic acid stimulation.

The experiments were carried out on human skin fragments obtained from elective blepharoplasty of healthy female patients. The explants were kept in culture and treatment with the sample was carried out on skin fragments from 3 different donors and in triplicate for each donor for a period of 24 hours.

The portion was subjected to total RNA extraction. cDNA was made from the RNA, which was subjected to a real time RT-PCR (Polymerase-Chain Reaction) reaction to assess the expression of 96 genes. Gene expression profiling and selection of differentially expressed genes were performed with the aid of Expression Suite Software v.1.0.3 (Life Technologies). ΔCt values for the reference and target genes were calculated by subtracting the values of the experimental groups. Subsequently, the ΔCt of the experimental group was subtracted from the control group (untreated skin explant) to obtain ΔΔCt. Finally, the relative quantification of target genes was determined by the equation: RQ = 2^-ΔΔCt. Only genes that presented a 1.3 threshold were selected, that is, 30% increase or decrease over the control. Statistical significance was assessed by the t-test accompanied by the Benjamini-Hochberg method (FDR - false discovery rate), with a p-value < 0.05 being considered significant.

### Example 2- Results of the screening on the impact on microbiota.

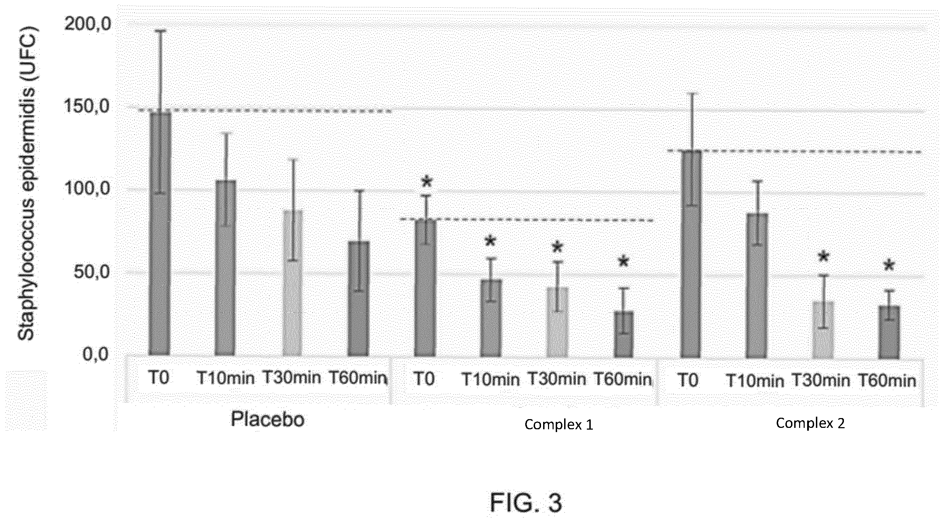

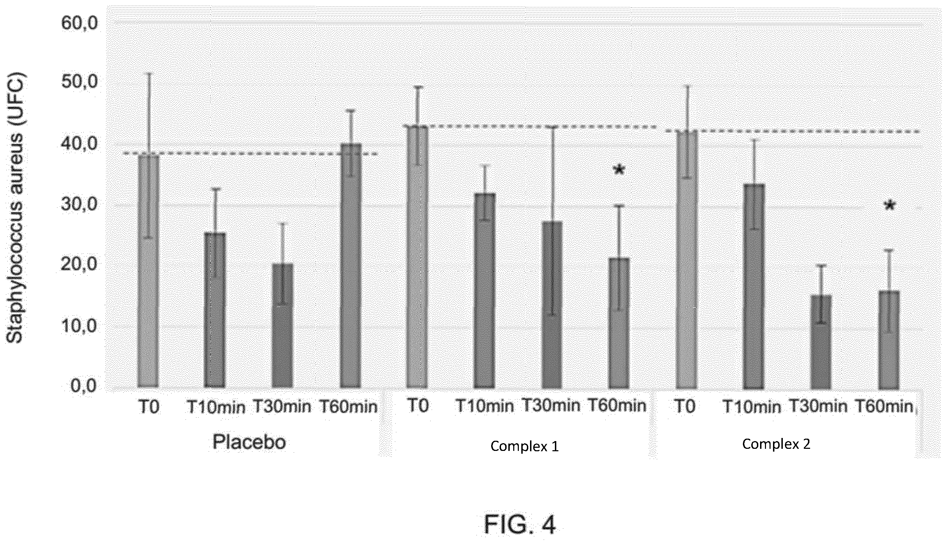

Figures 3 and 4 show the viability kinetics for *S. epidermidis and S***.** *aureus.*

In Figure 3, *S. epidermidis* viability kinetics is equivalent for the 3 samples. The count has dropped over time, but none of the samples made the presence of the beneficial organism unfeasible.

In figure 4, the placebo favored the recovery of *S. aureus* at T60. This is an unwanted feature, as *S. aureus* is involved in several skin pathologies. This effect did not happen when actives were added to both formulations..

Those skilled in the art will value the knowledge presented herein and may reproduce the invention in the disclosed embodiments and other variants, as covered by the scope of the appended claims.

## Claims

1. Cosmetic complex **characterized by** being an association comprising the following ingredients: *Fevillea trilobata* seed crude oil, trehalose and sodium hyaluronate.

2. The complex of claim 1, **characterized in that** it is an association comprising:
- 7% to 28% by weight *Fevillea trilobata* crude seed oil;
- 13% to 76% trehalose;
- 4% to 80% sodium hyaluronate.

3. The complex according to any one of claims 1 to 2, **characterized in that** it comprises betaine.

4. The complex according to any one of claims 1 to 3, **characterized in that** it comprises:
- 8% to 17% by weight *Fevillea trilobata* crude seed oil;
- 28% to 52% trehalose;
- 4% to 17% sodium hyaluronate;
- 34% to 43% betaine.

5. The complex according to any one of claims 1 to 4, **characterized in that** the association of *Fevillea trilobata* seed crude oil, trehalose, sodium hyaluronate and betaine is at a 2:12:1:8 ratio.

6. Use of the cosmetic complex as defined in any one of claims 1 to 5, **characterized in that** it is to hydrate the skin.

7. Use of the cosmetic complex as defined in any one of claims 1 to 5, **characterized in that** it is to preserve the skin natural microbiota.

8. Use of the cosmetic complex, as defined in any one of claims 1 to 5, **characterized in that** it is in the manufacture of a topical cosmetic product for skin hydration.

9. Use of the cosmetic complex as defined in any one of claims 1 to 5, **characterized in that** it is in the manufacture of a topical cosmetic product to preserve the skin natural microbiota.

10. Topical cosmetic product to hydrate and/or preserve the skin natural microbiota **characterized in that** it comprises a cosmetic complex as defined in any one of claims 1 to 5.

11. A method to hydrate and/or preserve the skin natural microbiota **characterized in that** it comprises applying the cosmetic complex, as defined in any one of claims 1 to 5 onto the skin.
